# EUROPEAN PATENT SPECIFICATION

(11) **EP 1 698 338 B1**
(45) Date of publication and mention of the grant of the patent: **09.04.2008**
(21) Application number: 06116051.1
(22) Date of filing: 28.05.2001
(51) Int. Cl.: A61K 31/496, A61P 25/18

(54) **S-methyl-dihydro-ziprasidone for the treatment of schizophrenia**
S-Methyl-Dihydro-Ziprasidone für die Behandlung von Schizophrenie
S-methyl-dihydro-ziprasidone pour traiter la schizophrénie

(30) Priority: 02.06.2000 US 209136 P; 16.06.2000 US 212172 P
(43) Date of publication of application: 06.09.2006
(62) Divisional of application: 01932011.8
(73) Proprietor: Pfizer Products Inc., Groton, CT 06340 (US)
(72) Inventor: Prakash, Chandra A., GrotonN, CT 06340 (US); Smolarek, Teresa A., Groton, CT 06340 (US)
(74) Representative: Duncan, Garreth Andrew

(56) References cited:
- WO-A-00/59489
- WO-A-97/42190
- WO-A-97/42191
- PRAKASH C ET AL: "METABOLISM AND EXCRETION OF A NEW ANTIPSYCHOTIC DRUG, ZIPRASIDONE, IN HUMANS" DRUG METABOLISM AND DISPOSITION, WILLIAMS AND WILKINS., BALTIMORE, MD, US, vol. 25, no. 7, July 1997 (1997-07), pages 863-872, XP000987000 ISSN: 0090-9556
- DANIEL D G ET AL: "Ziprasidone 80 mg/day and 160 mg/day in the acute exacerbation of schizophrenia and schizoaffective disorder: a 6-week placebo-controlled trial. Ziprasidone Study Group." NEUROPSYCHOPHARMACOLOGY : OFFICIAL PUBLICATION OF THE AMERICAN COLLEGE OF NEUROPSYCHOPHARMACOLOGY. MAY 1999, vol. 20, no. 5, May 1999 (1999-05), pages 491-505, XP002392761 ISSN: 0893-133X
- SEEGER T F ET AL: "Ziprasidone (CP-88,059): a new antipsychotic with combined dopamine and serotonin receptor antagonist activity." THE JOURNAL OF PHARMACOLOGY AND EXPERIMENTAL THERAPEUTICS. UNITED STATES OCT 1995, vol. 275, no. 1, October 1995 (1995-10), pages 101-113, XP002051730 ISSN: 0022-3565

## Description

### Background of the Invention

This invention relates to the use of S-methyl-dihydro-ziprasidone and its pharmaceutically acceptable salts in the manufacture of a medicament for the treatment of schizophrenia.

S-methyl-dihydro-ziprasidone, which has the following structure: 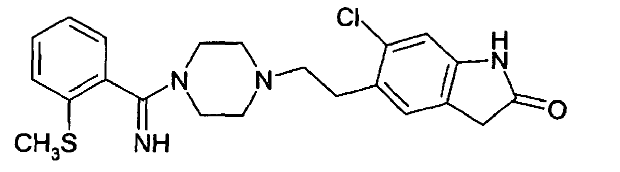 and the chemical name 6-chloro-5-(2-{4-[imino-(2-methylsulfanylphenylrmethyl]-piperazin-1-yl}-ethyl)-1,3-dihydro-indol-2-one, is an active metabolite of the antipsychotic drug ziprasidone, which has the following structure:

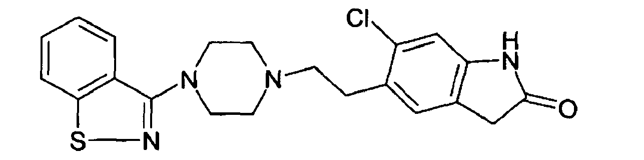

Ziprasidone and related arylpiperazinyl-(C₂-C₄)alkylene-heterocyclic compounds, methods for their synthesis and their use in the treatment of psychotic disorders of the schizophrenic types and for removing or ameliorating such symptoms as anxiety, agitation, excessive aggression, tension and social or emotional withdrawal in psychotic patients are referred to in United States Patent 4,831,031, which issued on May 16, 1989, United States Patent 5,206,366, which issued on April 27, 1993, United States Patent 4,833,795, which issued on November 28, 1989, United States Patent 5,312,925, which issued on May 17, 1994 and United States Patent 5,338,846, which issued on August 16, 1994. The use of ziprasidone and such related compounds for the treatment of obsessive-compulsive disorder and Tourette's syndrome is referred to in United States Patent Application 09/146,289, which was filed on September 3, 1998. The use of ziprasidone and such related compounds for the treatment of behavioral symptoms associated with psychosis is referred to in United States Patent Application 09/216,344, which was filed on December 8, 1998. The use of ziprasidone and such related compounds for the treatment of glaucoma and ischemic retinopathy is referred to in United States Patent Application 09/314,792, which was filed on May 18, 1999.

WO 00/59489, which was published after the priority dates of the present application, but before the filing date, describes methods of using, and compositions comprising, ziprasidone metabolites.

Prakash et al., Drug Met. Dis., (1997), 25(7), 863-872, describes the pharmacokinetics, metabolism, and excretion of ziprasidone in humans.

Daniel and Copeland, Exp. Opin. Invest. Drugs, 2000, 9(4), 819-828, provides an overview of ziprasidone and its clinical uses.

Daniel et al., Neuropsychopharmacology, (1999), 20, 491-505, describes the use of ziprasidone for treating schizophrenia.

WO 97/42190 describes ziprasidone mesylate trihydrate, pharmaceutical compositions containing it, and its use to treat psychotic diseases.

WO 97/42191 describes ziprasidone mesylate dihydrates, pharmaceutical compositions containing them, and their use to treat psychotic diseases.

### Summary of the Invention

This invention relates to the use of S-methyl-dihydro-ziprasidone, or a pharmaceutically acceptable salt thereof, in the manufacture of a medicament for treating schizophrenia in a mammal, including a human.

This use is hereinafter also referred to as "the inventive use".

The term "treating", as used herein, refers to reversing, alleviating, inhibiting the progress of, or preventing the disorder or condition to which such term applies, or one or more symptoms of such disorders or condition. The term "treatment", as used herein, refers to the act of treating, as "treating" is defined immediately above.

The present invention also relates to the above inventive use, wherein a radiolabelled form of S-methyl-dihydro-ziprasidone is used instead of S-methyl-dihydro-ziprasidone. Preferred radiolabelled compounds of S-methyl-dihydro-ziprasidone are those wherein the radiolabels are selected from as ³H, ¹¹C, ²H, ¹³C, ¹⁴C, ¹⁸F, ¹²³I and ¹²⁵I. Such radiolabelled compounds are useful as research and diagnostic tools in metabolism pharmacokinetics studies and in binding assays in both animals and man.

Examples of pharmaceutically acceptable acid addition salts of S-methyl-dihydro-ziprasidone are the salts of hydrochloric acid, p-toluenesulfonic acid, fumaric acid, citric acid, succinic acid, salicylic acid, oxalic acid, hydrobromic acid, phosphoric acid, methanesulfbnic acid, tartaric acid, maleic acid, di-p-toluoyl tartaric acid, acetic acid, sulfuric acid, hydroibdic acid and mandelic acid.

All the psychiatric disorders and conditions referred to herein are known to those of skill in the art and defined as in the Diagnostic and Statistical Manual of Mental Disorders, Fourth Edition, American Psychiatric Association, 1994 (DSM-IV).

### Detailed Description of the Invention

S-methyl-dihydro-ziprasidone can be prepared by reacting dihydro-ziprasidone with a methylating agent (e.g., iodomethane or diazomethane), preferably with iodomethane. This reaction is preferably carried out under a nitrogen atmosphere. It is typically carried out in a lower alkanol solvent such as methanol, ethanol or propanol, preferably methanol, at a temperature from about 0°C to about the reflux temperature of the solvent, preferably at about room temperature, in the presence of a strong base such as potassium hydroxide, sodium hydroxide, potassium or sodium t-butoxide, or potassium or sodium carbonate.

Dihydro-ziprasidone can be prepared as described in United States Patent 5,935,960, which issued on August 10, 1999.

The pharmaceutically acceptable acid addition salts of S-methyl-dihydro-ziprasidone are prepared in a conventional manner by treating a solution or suspension of the free base with about one chemical equivalent of a pharmaceutically acceptable acid. Conventional concentration and recrystallization techniques are employed in isolating the salts. Illustrative of suitable acids are the acetic, hydrochloric, hydrobromic, hydroiodic, nitric, sulfonic, sulfuric, isonicotinic, lactic, salicylic, citric, tartaric, pantothenic, bitartaric, ascorbic, succinic, maleic, fumaric, glucaronic, saccharate, formate, benzoate, glutamate, methanesulfonic, ethanesulfonic, benzenesulfonic, p-toluenesulfonic, and gluconic acids.

S-methyl-dihydro-ziprasidone and its pharmaceutically acceptable salts (referred to collectively, hereinafter, as "the active compounds of this invention"), can be administered to a human subject either alone, or, preferably, in combination with pharmaceutically acceptable carriers or diluents, in a pharmaceutical practice. Such compounds can be administered orally or parenterally. Parenteral administration includes especially intravenous and intramuscular administration. Additionally, in a pharmaceutical composition comprising an active compound of this invention, the weight ratio of active ingredient to carrier will normally be in the range from 1:6 to 2:1, and preferably 1:4 to 1:1. However, in any given case, the ratio chosen will depend on such factors as the solubility of the active component, the dosage contemplated and the precise route of administration.

The active compounds of this invention can be administered to mammals via either the oral, parenteral (such as subcutaneous, intravenous, intramuscular, intrastemal and infusion techniques), rectal, intranasal or topical routes. In general, these compounds are most desirably administered in doses ranging from about 0.5 to about 500 mg per day, in single or divided doses i.e., from 1 to 4 doses per day), although variations will necessarily occur depending upon the species, weight and condition of the subject being treated and the particular route of administration chosen. However, a dosage level that is in the range of about 10 mg to about 80 mg per kg of body weight per day is most desirably employed. Nevertheless, variations may occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such higher dose levels are first divided into several small doses for administration throughout the day.

The active compounds of this invention can be administered alone or in combination with pharmaceutically acceptable carriers or diluents by any of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral use in treating the various disorders and conditions referred to above, the can be administered, for example, in the form of tablets or capsules, or as an aqueous solution or suspension. Tablets containing various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

For intramuscular, parenteral and intravenous use, sterile solutions of the active ingredient can be prepared, and the pH of the solutions should be suitably adjusted and buffered. For intravenous use, the total concentration of solutes should be controlled to render the preparation isotonic.

Additionally, it is also possible to administer the compounds of the present invention topically when treating ocular disorders such as glaucoma and ischemic retinopathy and this may be done by way of creams, jellies, gels, pastes, patches, ointments and the like, in accordance with standard pharmaceutical practice.

The following example illustrates the synthesis of S-methyl-dihydro-ziprasidone.

### EXAMPLE 1

### 6-CHLORO-5-(2-{4-[IMINO-(2-METHYLSULFANYLPHENYL)-METHYL]-PIPERAZIN-1-YL}-ETHYL)-1,3-DIHYDRO-INDOL-2-ONE

Potassium hydroxide (1.82 grams) was added to methanol (1300 ml) under a nitrogen atmosphere and stirred for 15 minutes at 21°C. Dihydro-ziprasidone (13.0 grams, 31.3 mmol) was added and the mixture was stirred at 21°C until a solution was formed. lodomethane (2.34 ml, 37.7 mmol, 1.2 equivalents) was added and the solution was allowed to stir overnight at 21°C. The progress of the reaction was followed using thin layer chromatography (silica gel, eluting with 4:1 methylene chloride:isopropanol, visualizing with UV lamp at 254 nm). The reaction mixture was vacuum concentrated and 2.0 liters of methylene chloride and 500 ml of water were added to the remaining solid. The hazy mixture was allowed to stir for 15 minutes, after which the water layer (pH=9.8) was discarded. The methylene chloride layer, which was light pink, was dried using magnesium sulfate and Darco G60 was added to decolorize the solid. The mixture was allowed to stir for another 15 minutes at 21 °C and filtered over Celite. The Celite filter pad was washed with 50 ml methylene chloride and the combined filtrates were vacuum concentrated to a solid (13.05 grams) that was a lighter pink. Further purification using flash chromatography with silica gel and a methylene chloride eluent yielded 7.38 grams of the title compound. M.P. 103 - 106°C (gassing). Mass spectrum (m/e, % intensity): 429 (100, M+1), 236,194. ¹³C NMR (CDCI3) 177.70, 166.83, 142.36, 136.94, 136.17, 133.10, 131.30, 129.51, 126.86, 125.27, 124.46, 111.06, 58.84, 53.11, 36.09, 30.95 and 15.87.

## Claims

1. The use of 6-chloro-5-(2-{4-[imino-(2-methylsulfanylphenyl)-methyl]-piperazin-1-yl}-ethyl)-1,3-dihydro-indol-2-one, or a pharmaceutically acceptable salt thereof, in the manufacture of a medicament for treating schizophrenia in a mammal.

## Patentansprüche

1. Verwendung von 6-Chlor-5-(2-{4-[imino-(2-methylsulfanylphenyl)-methyl]-piperazin-1-yl}-ethyl)-1,3-dihydro-indol-2-on oder eines pharmazeutisch verträglichen Salzes davon zur Herstellung eines Medikaments zur Behandlung von Schizophrenie bei einem Säugetier.

## Revendications

1. Utilisation de 6-chloro-5-(2-{4-[imino-(2-méthyl-sulfanylphényl)-méthyl]-pipérazine-1-yl}-éthyl)-1,3-dihydro-indole-2-one, ou d'un de ses sels pharmaceutiquement acceptables, dans la production d'un médicament destiné au traitement de la schizophrénie chez un mammifère.
